# EUROPEAN PATENT SPECIFICATION

(11) **EP 4 436 638 B1**
(45) Date of publication and mention of the grant of the patent: **03.12.2025**
(21) Application number: 22826837.1
(22) Date of filing: 23.11.2022
(51) Int. Cl.: A61M 11/00, A61M 16/04

(54) **TRACHEAL ADMINISTERING DEVICE AND ATOMIZING CATHETER**
TRACHEALE VERABREICHUNGSVORRICHTUNG UND ZERSTÄUBUNGSKATHETER
DISPOSITIF D'ADMINISTRATION TRACHÉALE ET CATHÉTER DE PULVÉRISATION

(30) Priority: 23.11.2021 NL 2029874
(43) Date of publication of application: 02.10.2024
(73) Proprietor: Medspray B.V., 7521 PV Enschede (NL)
(72) Inventor: VAN RIJN, Cornelis Johannes Maria, 7521 PV Enschede (NL); HUIJGEN, Tom Vincent, 7521 PV Enschede (NL); VAN EGMOND, Henri Joseph, 7521 PV Enschede (NL)
(74) Representative: Jilderda, Anne Ayolt
(86) International application number: PCT/IB2022/061333
(87) International publication number: WO 2023/095019

(56) References cited:
- WO-A1-2008/056986
- US-A1- 2004 084 049

## Description

The present invention relates to a tracheal administering device for a liquid, comprising an endotracheal tube with a ventilation lumen which extends axially therein and is able and configured to carry a respiratory gas flow, and comprising a liquid conduit which connects proximally to a liquid system able and configured to supply a liquid under an increased operating pressure and from which the liquid is receivable into the respiratory gas flow distally.

Respiratory ventilation is used to support or replace the natural respiratory function of a patient. This patient is usually suffering from acute or chronic respiratory failure and is therefore unable to maintain their breathing independently. The treatment generally enables respiration by mechanically providing an oxygen-containing respiratory gas with a volume flow rate, humidity and pressure which are adapted to the patient. For this purpose the patient is connected to a respiratory device via an endotracheal tube which is inserted through the nose or mouth (tracheal intubation) or is introduced directly into the trachea via a surgically created opening (tracheostomy). The respiratory gas can then be administered via the endotracheal tube and be evacuated on an exhale.

It is often desirable here for one or more pharmaceutically active substances to be administered as well. For an optimal effectiveness it is preferred for such a substance to be administered directly in the lungs instead of for instance parenterally or otherwise.

The medicine thus goes directly to the location where it must act. Because it finds its way directly to the site of action, a lower dosage can be used. This decreases the chances of side effects. The medicine further has a rapid action. These substances are for instance anti-inflammatories and bronchodilators. Such medications are typically supplied in liquid form by the manufacturer. With a view to tracheal administration, the medicine must therefore first be converted into an aerosol of small droplets before the medicine can be administered to the lungs of the patient.

For conversion of a liquid medication into an aerosol for the purpose of tracheal administration use is in International patent application WO 2012/073236 made of an atomizer which is connected via a ventilator tube to the endotracheal tube and is incorporated in the ventilator externally of the patient. This known atomizer comprises a container in which a measured quantity of the medicine in liquid form is received. The liquid product is atomized in the atomizer and the aerosol formed thereby is mixed with the respiratory gasses flowing from the ventilator to the patient. Together therewith, the medicine reaches the lungs of the patient after having travelled a distance of several decimetres to several metres through the ventilator tube and the endotracheal tube.

Research has shown that in the case of such administration of a pharmaceutical agent a large part thereof precipitates against the walls of the ventilator tube and/or endotracheal tube. This can be as much as 50% to 90% of the atomized quantity. Not only does this result in a reduced efficiency and a reduced effectiveness of the medicine; it also makes the actual dosage of the medicine highly inaccurate and uncertain.

It is known from American patent US 7,469,700 to apply for the purpose of tracheal administration of a medication a special atomizing catheter which is inserted into the respiratory system and which provides a mist of the relevant medication at a distal tip thereof. For this purpose the catheter comprises a liquid channel through which the liquid medication is guided in combination with a gas tube which carries a gas under increased pressure to the distal tip of the liquid channel. The gas which finally escapes at the outer end collides with the liquid, which will thereby burst into a mist and will be entrained by the respiratory air.

A drawback of this known atomizing catheter is that the liquid mist formed thereby is relatively uncontrollable and thereby propagates in the ventilation lumen in more or less unpredictable manner. A part of the formed mist will still precipitate onto the wall of the endotracheal tube or the wall of the trachea without ever reaching the patient's lungs. WO2008/056986 describes assemblies of of an atomizer body and respiratory tube.

The present invention has for its object, among others, to provide a tracheal administering device for an active substance which provides increased control of the actual dosage and efficacy of the introduced medicine.

In order to achieve the stated object a tracheal administering device of the type described in the preamble has the feature according to the invention that the liquid conduit is distally coupled to a spraying device comprising a ceramic spray plate with at least one spray channel, that the at least one spray channel has a cross-section smaller than about five micrometres, and that the at least one spray channel receives the liquid, at least during operation, from the liquid conduit under increased pressure and delivers therefrom at least one mist jet to the respiratory gas flow. The at least one mist jet comprises a series of droplets of at least substantially the same size into which the liquid has been scattered as a result of Rayleigh instability, this size being in the order of at most twice to three times the stated cross-section of the spray channel.

The mist is thus formed directly from the liquid by means of Rayleigh scattering in or at least close to the microscopic channel opening through which the liquid was forced under pressure. This process is characterized by an almost mathematical, i.e. physically imposed, precision with which a jet of successive droplets of substantially identical shape and size is formed from the liquid. This size lies at most between twice to three times the diameter of the channel from which the droplets escape. It is noted here that, where reference is made in the present application to a size or diameter, this is always understood to mean, unless explicitly stated otherwise, the corresponding diameter of a circle or sphere with the same active section or volume as for instance the channel or the drop, if they were not purely or substantially round or spherical.

Owing to the imposed precision in the formed aerosol cloud, all droplets therein behave substantially uniformly and predictably. A choice of the channel diameter determines here the individual droplet size. The interaction with the respiratory gas flowing past is thereby predictable and so is the distance over which the aerosol droplets will be entrained. The delivered dosage can then also be administered effectively and accurately with a high degree of certainty.

In order to avoid irritation of the airways and in order not to disrupt an endoluminal tracheal introduction of the device, a preferred embodiment of the device has the feature according to the invention that at least the distal part of the liquid conduit extends inside the endotracheal tube and particularly ends at least substantially concentrically therein. The external contour (OD) of the device is thus not affected, at least in the relevant part, by the presence of the liquid channel.

For manufacture of the spraying device and particularly the spray plate use is advantageously made of photolithographic masking and etching techniques, as have become standard in modern semiconductor technology. With a view thereto, a further preferred embodiment of the device has the feature according to the invention that the ceramic spray plate comprises a silicon nitride layer in which the spray opening extends over a whole layer thickness, that the silicon nitride layer extends over a silicon carrier body with a body cavity which opens at the position of the spray channel at a surface thereof, and that the body cavity maintains a liquid connection to the distal outer end of the liquid conduit. The spray device thus comprises essentially only or at least mainly materials which are compatible with silicon semiconductor technology. The dimensions, form and location of the spray channels can thereby be defined extremely precisely using photolithography and be realized within microscopic to sub-micron tolerances.

In a particular embodiment the device according to the invention has the feature that the endotracheal tube comprises an inlet tube with a port through which the liquid conduit is receivable, which inlet tube continues in the ventilation lumen of the endotracheal tube and, within a distance of 10 centimetres upstream of the distal outer end of the endotracheal tube, opens therein. The spray device thus delivers inside the endotracheal tube a mist which will be entrained therein by a mainly unidirectional gas flow of the respiratory gas. Divergence of the mist is hereby counteracted, and thereby deposition thereof against the wall of the endotracheal tube or the trachea downstream thereof. In order to fully utilize this effect, a preferred embodiment of the device has the feature here that at least an outer end of the inlet tube is received by a centering device, which centering device centres at least the outer end of the inlet tube substantially coaxially in the ventilation lumen.

In order to limit the operating pressure of the spraying device so that, if desired, it can also be produced manually, a further particular embodiment of the spraying device has the feature according to the invention that the at least one spray channel has a length of less than about five micrometres.

In a further particular embodiment the device has the feature according to the invention that the endotracheal tube comprises close to the distal open outer end thereof an optical image recorder which is couplable to an image processing device and screen. During use of the device the spray pattern can thus be monitored and blockage or contamination of the spraying device can for instance be determined. Suitable action can then be taken.

With a view to possible cleaning of the spraying device a further particular embodiment of the device has the feature according to the invention that the endotracheal tube comprises a suction tube with a suction nozzle which opens close to the spraying device, and that the endotracheal tube comprises a port with a tube connection for connecting the suction tube to an external suction device. The suction tube enables interim removal by the user of any deposit of mucus in particular from the spraying device, without having to take out the spraying device and/or the endotracheal tube for this purpose. This ensures a continued operational reliability and readiness of the device.

Although the liquid system can be embodied in numerous ways, a particularly practical and simple yet no less effective embodiment of the device has the feature according to the invention that the liquid system comprises a medical syringe comprising a container with a volume for the liquid in which a manually energizable piston body is moveable, and that the liquid conduit comprises at the proximal outer end a coupling for a liquid-tight and pressure-resistant connection to an outlet of the medical syringe. A medical syringe is here understood to mean the liquid-containing part of a commonly available and generally used medical syringe without hypodermic needle. The spout thereof forms the outlet which is connected to the liquid tube directly or via a suitable coupling, while the syringe comprises the medicine to be administered. It has been found that an operating pressure in the order of 10 bar can be manually produced thereby, this being sufficient for an optimal operation of the spraying device.

In a preferred embodiment the device has the feature according to the invention that the liquid conduit is provided by an individual atomizing catheter comprising an elongate shaft body in which a liquid lumen extends axially with a distal outer end which connects to the spraying device, wherein the atomizing catheter is received exchangeably in the endotracheal tube. The application of such an individual and exchangeable atomizing catheter in the endotracheal tube has numerous advantages, which will be elucidated below on the basis of a specific exemplary embodiment.

The invention also relates to such an individual atomizing catheter and will be further elucidated hereinbelow with reference to an exemplary embodiment and an accompanying drawing. In the drawing:
- Figure 1: is an exemplary embodiment of a tracheal administering device according to the invention;
- Figure 2: is a detail section of the spraying device as applied in the device of figure 1;
- Figure 3: is a cross-section through the spray plate as applied in the spraying device of figure 2; and
- Figure 4: is a front view of the spraying device of figure 2.

It is otherwise noted here that the figures are purely schematic and not always drawn to (the same) scale. Some dimensions in particular may be exaggerated to greater or lesser extent for the sake of clarity. Corresponding parts are designated in the figures with the same reference numeral.

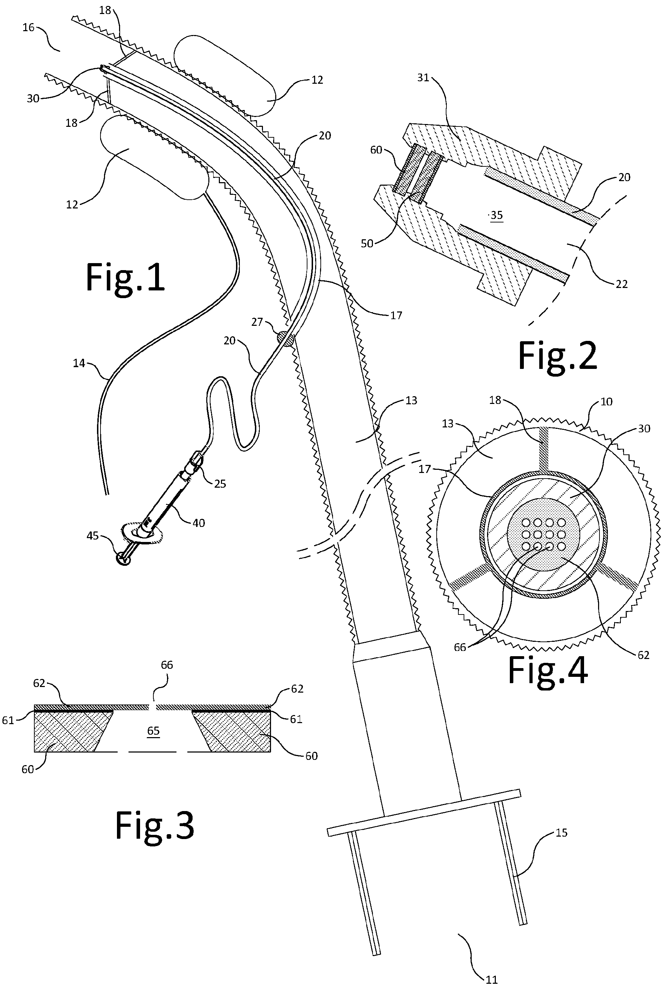

Figure 1 shows an embodiment of an administering device according to the present invention. Figure 1 shows a flexible endotracheal tube 10 of a suitable plastic, which can be an endotracheal tube available as standard or, as it is here, can be adapted especially for use with the spraying device according to the invention. Inside the endotracheal tube 10 a ventilation lumen 13 extends from a proximal outer end 11 to an open distal outer end 16 of the endotracheal tube. The endotracheal tube 10 has at a proximal outer end a standard connection 15 for connection to a ventilator circuit of a ventilator (not further shown). By means of this ventilator a respiratory airflow, which is carried to the patient via the ventilation lumen 13, can be maintained artificially.

The endotracheal tube 10 has in a distal part an inflatable collar 12 which lies close to its distal outer end in order to facilitate positioning of tube 10 in the trachea of the patient. The inflatable collar 12 is connected via a separate inflatable lumen to a proximal fitting for connection to a source of inflating gas (not shown). The inflatable lumen can be integrated in the endotracheal tube or, as it is here, be provided by a separate inflatable catheter 14. The inflatable lumen extends therethrough in order to carry an inflating gas or other pressure medium from means provided for this purpose to the inside of collar 12. Collar 12 is thus inflatable in vivo in order to close a space around the endotracheal tube in the trachea to a parasitic airflow.

Provided inside the endotracheal tube, over at least a part of a length thereof, is an inlet tube 17 through which a liquid conduit 20 can be introduced into the ventilation lumen 13. In this embodiment the liquid conduit is formed by an individual atomizing catheter 20 with a shaft through which a liquid lumen 22 extends axially over a whole length. A liquid with an active substance therein can be guided via liquid lumen 22 to the distal outer end of the liquid conduit under increased pressure in the order of 10 to 20 atmosphere. At the distal outer end the atomizing catheter comprises a spraying device 30 which will be discussed in further detail below and which forms a fine mist from the liquid and delivers this mist to the surrounding area.

The inlet tube 17 extends in the ventilation lumen over at least a part of the length thereof and is supported at the outer end by a centering device 18, see also figure 4. This centering device ensures that a distal outer end of inlet tube 17 opens substantially co-axially centrally in ventilation lumen 13. Figure 1 shows the endotracheal tube 10 in a situation where the atomizing catheter 20 is carried therethrough and protrudes conversely with the spraying device 30 outside the inlet tube 17. Spraying device 30 thereby lies substantially centrally in ventilation lumen 13.

Situated at a proximal outer end of atomizing catheter 20 is a connection for a liquid system. In this embodiment this is a standard connection, such as a Luer fitting, to which a standard medical syringe 40 can be connected. The construction of medical syringe with a transparent liquid reservoir therein is assumed sufficiently known and is eminently suitable for supplying the active liquid under the increased operating pressure in the order of 10 to 20 atmosphere. For this purpose the plunger 45 is pushed in manually and the administered dosage can be read in the wall of the transparent reservoir.

In this exemplary embodiment use is made of an individual atomizing catheter 20 which is provided removably and replaceably in the endotracheal tube 10. This provides several significant advantages. Firstly, the atomizing catheter can be specifically adapted and selected to have the desired operating characteristics which are suitable for delivering the specific medication which is administered to the patient. The fact that the atomizing tube is removable and replaceable furthermore provides versatility and flexibility in respect of the therapy and the dosage regimen that can be opted for by the doctor. A decision on the part of the doctor to administer for instance a medication to the airways and the choice of the type and the dosage of the medicine to be administered need only be made by the doctor once the endotracheal tube has been inserted in the patient.

When the doctor determines the correct type of medication to be administered to the patient via the airways, a corresponding atomizing catheter can be selected and can be introduced via the endotracheal tube. The atomizing catheter can further be removed in simple manner after use and therefore preferably does not remain in the patient any longer nor takes up space in the respiratory tract of the patient or in endotracheal tube 10 once it is no longer needed. The decision regarding the correct type of medication can furthermore be reassessed at any moment after the endotracheal tube has been placed. If a different type of atomizing catheter is needed, such as for instance from a viewpoint of sterility purposes or local anaesthesia, the endotracheal tube 10 need not be replaced as well.

Another advantage of providing the atomizing catheter as an individual, removable apparatus is that it can be incorporated in a diversity of other instruments and/or apparatuses. The atomizing catheter is used in endotracheal tube; the atomizing catheter could however also be placed in a bronchoscope, such as in an operative channel of a bronchoscope. The atomizing catheter could be placed in any instrument which is placed in the respiratory tract and which is suitable for the size of the atomizing catheter.

Atomizing catheter 20 can be provided with radiopaque markers 25, see figure 2, in order to facilitate its positioning and placing and to enable it to be tracked on a screen. Use is then made for this purpose of radiographic equipment which is coupled to the screen. The radiopaque markers 25 can be provided by radiopaque strips of a metal or by heat-shrunk strips of doped radiopaque plastic which are attached to the shaft of the atomizing catheter. Such markers can also be arranged in numerous other ways and positions by doping the plastic material of the atomizing catheter with a radiopaque material. Alternatively, a radiopaque contrast dye can be added to the liquid which is delivered by the atomizing catheter in order to facilitate observation. The markers 25 can be graded in order to facilitate recognition, or can alternatively extend over a part of or the whole atomizing catheter. The markers can also be formed by ultrasonic reflectors, for instance in the form of a structured material, which is visible by means of ultrasonic imaging. The atomizing catheter can also comprise a stripe which extends along a side of the shaft and which is radiopaque or ultrasonically visible and then also provides an indication of a rotation orientation of the shaft of the catheter 20. The stripe can be formed by a co-extrusion or by embedding a wire in the wall of the atomizing catheter.

The atomizing catheter can also comprise a safety stop 27 which is situated along a proximal portion and runs up against the wall of the endotracheal tube, see figure 1. In this way the doctor can control the final positioning of the spraying device in the ventilation lumen. The safety stop 27 ensures that the distal outer end of atomizing catheter 20 is positioned correctly relative to the distal outer end 16 of endotracheal tube 10 and prevents the distal outer end of the atomizing catheter from protruding too far into the patient's trachea. In addition to the safety stop 27, the proximal part of atomizing catheter 20 can also have distance markers which are visible to the doctor and which provide an indication of the distance over which the atomizing catheter was inserted and thereby of the position of the distal outer end of atomizing catheter 20 relative to a distal outer end 46 of endotracheal tube 10.

If desired, use can also be made of an atomizing catheter around which a suction tube extends co-axially, which are then inserted together via inlet tube 17. In that case a coaxial suction lumen lies between the atomizing catheter and the inner wall of the suction tube. An external suction device can then be connected thereto proximally, whereby an underpressure can be generated and maintained therein. Any deposition, for instance of mucus, on spraying device 20 can thereby be suctioned away, and the suction tube can optionally be manipulated past this point in order to also be able to rid the trachea and/or bronchi of lung mucus or the like downstream.

If desired, such a suction tube can be connected proximally via a manifold together with the atomizing catheter. In that case such a manifold also comprises a gas port for the suction system, this in addition to a liquid port to which the liquid system, such as here the medical syringe 40, can be connected. These ports 28 and 32 can comprise conventional fastening means, such as fittings of the luer lock type. These ports can furthermore also be provided with closing caps which can be used to close the ports when they are not in use and which can be opened when connection to a suction installation or the liquid system is desired. The manifold can optionally comprise a filter which is placed in line with the liquid port in order to prevent blockages of the liquid lumen as a result of solid particles present therein.

The length of atomizing catheter 20 must be sufficient for spraying device 30 to be placed at the desired location sufficiently far into the respiratory system, while the proximal outer end with coupling 25 is accessible to the doctor or other medical staff for connection to the liquid system 40 outside the body of the patient. Accordingly, the length of atomizing catheter 20 depends on the size of the patient in which it is being used. A shorter atomizing catheter can be preferred for smaller patients, such as infants or children, and a longer atomizing catheter may be necessary for adults. An atomizing catheter 20 suitable for adults can for instance have a length of about 45 cm. In this embodiment about 30 cm of atomizing catheter 20 is situated in the endotracheal tube 10. The atomizing catheter can otherwise also be used to deliver an aerosol of the liquid with the active substance to the nasal cavities and/or sinus cavities of a patient, in which case the length can be correspondingly shorter.

Figure 2 shows the distal outer end of atomizing catheter 20 with the spraying device 30 thereon. The spraying device comprises a plastic adapter body 31 in which the distal shaft part 20 of the atomizing catheter is fittingly received such that the liquid lumen 22 extending therein opens directly into a body cavity 35 which is provided in the adapter body 31. For the purpose of a reliable, robust mutual connection the two parts 20, 35 are glued or welded to each other so that pressure which is significantly greater than the operating pressure of the liquid to which the connection may be exposed during operation can be withstood.

Situated successively in the body cavity are a filter unit 50 and a spraying unit 60. The filter unit can be formed by a porous body or, as it does here, comprise a silicon semiconductor body with a top layer in which a large number of microscopic passages have been etched using photolithography. For this purpose use can be made of standard modern semiconductor technology, and existing micro-machining techniques also provide ample possibility therefor.

The spraying unit, which in this embodiment is accommodated in the spraying device, is shown in further detail in figure 3. Use is made therefor of a semiconductor body 60 of silicon which is covered with, successively, a stress-reducing silicon oxide layer 61 and ceramic silicon nitride layer 62 so as to form a spray plate therefrom. The silicon oxide layer 61 can be realized with a thickness of half a micron to one micron by thermal oxidation of the silicon body 60. The silicon nitride layer 62 is deposited thereon with a thickness in the order of one or several microns, for instance by means of chemical vapour deposition, CVD for short. In this embodiment the silicon nitride layer is 2 microns thick.

The ceramic silicon nitride layer 62 spans a body cavity 65 in the semiconductor body 60 which was etched locally therein from below using photolithography. At the position of body cavity 65 a spray channel 66with a diameter of a maximum of 5 microns is etched through the nitride layer 62 over a whole thickness with great precision. In this embodiment the spray channel 66 comprises a substantially cylindrical passage with a diameter of 1 micron. More spray channels 66, which may or may not be identical, can optionally be provided per body cavity 65, and more body cavities, each with one or more spray channels 66, can also be provided in the semiconductor body 60. A ceramic spray plate 62 of silicon nitride thus lies on silicon body 60.

For the oxidation for forming oxide layer 61, and for the deposition of nitride layer 62, and for etching of body cavity 65 and for forming the passage 66 use is made of modern semiconductor technology whereby fixedly defined patterns and structures can be realized on a sub-micron scale with very high precision and reproducibility. This makes it possible to set and predict the spraying behaviour and character of spraying device 30 with very high precision. The dimensions are thereby selected such that the formed mist will be entrained optimally in the ventilation flow.

Both the filter unit 50 and the spraying unit 60 are anchored liquid-tightly all around in the body cavity 35 of adapter body 31. The liquid which is supplied through liquid conduit 20 and received in body cavity 35 under an operating pressure of 10 to 20 atmosphere forces its way via filter unit 50 and body cavity or cavities 65 in spraying unit 60 to the ceramic top layer 62 with the spray channels 66 therein, see also figure 4. In or directly downstream of spray channel 66 the liquid flow becomes unstable and scatters into successive droplets. The position of scattering is physically predetermined in the applied dimensions and materials of the device, whereby a strictly defined scattering occurs, this resulting in droplets of substantially identical size. In practice, this uniform scattering results in a droplet diameter of between twice and three times the diameter of spray channel 66.

Figure 4 shows a front view of the spraying device with a number of such spray channels 60 whereby a substantially monodisperse mist can thus be formed in particularly precise manner. Owing to the small droplet size, as it were a gas, this mist is able to be entrained by the respiratory gas flow in ventilation lumen 13 so as to land substantially wholly in the bronchi where the active substance in the liquid is intended to end up. A particularly precise dosing is thus possible, which was not achievable with the existing means.

Although the invention has been further elucidated above with reference to only a single exemplary embodiment, it will be apparent that the invention is by no means limited thereto. On the contrary, many variations and embodiments are still possible within the scope of the invention for a person with ordinary skill in the art. Use is in this embodiment thus made of an individual atomizing catheter to provide a liquid conduit, but such a conduit can also be formed by a liquid lumen forming part of the endotracheal tube.

## Claims

1. Tracheal administering device for a liquid, comprising an endotracheal tube (10) with a ventilation lumen (13) which extends axially therein and is able and configured to carry a respiratory gas flow, and comprising a liquid conduit (20) which connects proximally to a liquid system able and configured to supply a liquid under an increased operating pressure and from which the liquid is receivable into the respiratory gas flow distally, wherein the liquid conduit (20) is distally coupled to a spraying device (30) comprising a ceramic spray plate (60) with at least one spray channel (66) that has a cross-section smaller than about five micrometers, and wherein the at least one spray channel receives the liquid, at least during operation, from the liquid conduit under increased pressure and delivers therefrom at least one mist jet to the respiratory gas flow, **characterized in that** the endotracheal tube comprises an inlet tube (17) with a port through which the liquid conduit (20) is receivable, which inlet tube continues in the ventilation lumen (13) of the endotracheal tube and opens within a distance of 10 centimetres upstream of the distal outer end (16) of the endotracheal tube, **in that** at least an outer end of the inlet tube is received by a centering device (18) that centres at least the outer end of the inlet tube substantially coaxially in the ventilation lumen (13), **in that** the liquid conduit is provided by an individual atomizing catheter (20) comprising an elongate shaft body in which a liquid lumen extends axially, having a distal outer end which connects to the spraying device (30), and **in that** the atomizing catheter is received exchangeably in the endotracheal tube (10).

2. Device according to claim 1, **characterized in that** at least the distal part of the liquid conduit (20) extends inside the endotracheal tube (10) and particularly ends at least substantially concentrically therein.

3. Device according to claim 1 or 2, **characterized in that** the ceramic spray plate (60) comprises a silicon nitride layer (62) in which the spray opening (66) extends over a whole layer thickness, that the silicon nitride layer extends over a silicon carrier body (60) with a body cavity (65) which opens at the position of the spray channel at a surface thereof, and that the body cavity maintains a liquid connection to the distal outer end of the liquid conduit (20).

4. Device according to one or more of the preceding claims, **characterized in that** the at least one spray channel (66) has a length of less than about five micrometers.

5. Device according to one or more of the preceding claims, **characterized in that** the endotracheal tube (10) comprises close to the distal open outer end thereof an optical image recorder which is couplable to an image processing device and screen.

6. Device according to one or more of the preceding claims, **characterized in that** the endotracheal tube (10) comprises a suction tube with a suction nozzle which opens close to the spraying device, and that the endotracheal tube comprises a port with a tube connection for connecting the suction tube to an external suction device.

7. Device according to one or more of the preceding claims, **characterized in that** the liquid system comprises a medical syringe (40) comprising a container with a volume for the liquid in which a manually energizable piston body (45) is moveable, and that the liquid conduit comprises at the proximal outer end a coupling (25) for a liquid-tight and pressure-resistant connection to an outlet of the medical syringe.

## Patentansprüche

1. Tracheale Verabreichungsvorrichtung für eine Flüssigkeit, umfassend einen Endotrachealtubus (10) mit einem Ventilationslumen (13), das sich axial darin erstreckt und geeignet ist und ausgebildet ist, einen Atemgasstrom zu führen, und umfassend eine Flüssigkeitsleitung (20), die proximal mit einem Flüssigkeitssystem verbunden ist, das geeignet und ausgebildet ist, eine Flüssigkeit unter einem erhöhten Betriebsdruck zuzuführen, und aus der die Flüssigkeit distal in den Atemgasstrom aufnehmbar ist, wobei die Flüssigkeitsleitung (20) distal mit einer Sprühvorrichtung (30) gekoppelt ist, die eine keramische Sprühplatte (60) mit mindestens einem Sprühkanal (66) umfasst, der einen Querschnitt kleiner als etwa fünf Mikrometer aufweist, und wobei der mindestens eine Sprühkanal die Flüssigkeit zumindest im Betrieb aus der Flüssigkeitsleitung unter erhöhtem Druck aufnimmt und daraus mindestens einen Nebelstrahl an den Atemgasstrom abgibt, **dadurch gekennzeichnet, dass** der Endotrachealtubus ein Einlassrohr (17) mit einer Öffnung umfasst, durch die die Flüssigkeitsleitung (20) aufnehmbar ist, wobei sich das Einlassrohr in das Ventilationslumen (13) des Endotrachealtubus fortsetzt und innerhalb eines Abstands von 10 Zentimetern stromaufwärts des distalen äußeren Endes (16) des Endotrachealtubus mündet, ferner dadurch, dass zumindest ein äußeres Ende des Einlassrohrs von einer Zentriervorrichtung (18) aufgenommen ist, die zumindest das äußere Ende des Einlassrohres im Wesentlichen koaxial im Ventilationslumen (13) zentriert, ferner dadurch, dass die Flüssigkeitsleitung durch einen individuellen Zerstäubungskatheter (20) bereitgestellt wird, der einen länglichen Schaftkörper umfasst, in dem sich ein Flüssigkeitslumen axial erstreckt, der ein distales äußeres Ende aufweist, das mit der Sprühvorrichtung (30) verbunden ist, und dass der Zerstäubungskatheter austauschbar im Endotrachealtubus (10) aufgenommen wird.

2. Vorrichtung nach Anspruch 1, **dadurch gekennzeichnet, dass** zumindest der distale Teil der Flüssigkeitsleitung (20) innerhalb des Endotrachealtubus (10) verläuft und insbesondere zumindest im Wesentlichen konzentrisch darin endet.

3. Vorrichtung nach Anspruch 1 oder 2, **dadurch gekennzeichnet, dass** die keramische Sprühplatte (60) eine Siliziumnitridschicht (62) aufweist, in der sich die Sprühöffnung (66) über eine ganze Schichtdicke erstreckt, dass sich die Siliziumnitridschicht über einen Siliziumträgerkörper (60) mit einem Körperhohlraum (65) erstreckt, der an der Stelle des Sprühkanals an einer Oberfläche desselben mündet, und dass der Körperhohlraum eine Flüssigkeitsverbindung zum distalen äußeren Ende der Flüssigkeitsleitung (20) aufrechterhält.

4. Vorrichtung nach einem oder mehreren der vorhergehenden Ansprüche, **dadurch gekennzeichnet, dass** der mindestens eine Sprühkanal (66) eine Länge von weniger als etwa fünf Mikrometer aufweist.

5. Vorrichtung nach einem oder mehreren der vorhergehenden Ansprüche, **dadurch gekennzeichnet, dass** der Endotrachealtubus (10) in der Nähe seines distalen offenen äußeren Endes einen optischen Bildrekorder aufweist, der mit einer Bildverarbeitungseinrichtung und einem Bildschirm koppelbar ist.

6. Vorrichtung nach einem oder mehreren der vorhergehenden Ansprüche, **dadurch gekennzeichnet, dass** der Endotrachealtubus (10) einen Saugschlauch mit einer Saugdüse aufweist, die nahe der Sprühvorrichtung mündet, und dass der Endotrachealtubus einen Anschluss mit einem Schlauchanschluss zum Verbinden des Saugschlauchs mit einer externen Saugvorrichtung aufweist.

7. Vorrichtung nach einem oder mehreren der vorhergehenden Ansprüche, **dadurch gekennzeichnet, dass** das Flüssigkeitssystem eine medizinische Spritze (40) umfasst, die einen Behälter mit einem Volumen für die Flüssigkeit aufweist, in dem ein manuell erregbarer Kolbenkörper (45) bewegbar ist, und dass die Flüssigkeitsleitung am proximalen äußeren Ende eine Kupplung (25) für eine flüssigkeitsdichte und druckfeste Verbindung mit einem Auslass der medizinischen Spritze aufweist.

## Revendications

1. Dispositif d'administration trachéale d'un liquide, comprenant un tube endotrachéal (10) avec une lumière de ventilation (13) qui s'étend axialement à l'intérieur et qui est capable et configurée pour transporter un flux de gaz respiratoire, et comprenant un conduit de liquide (20) qui se connecte proximalement à un système de liquide capable et configuré pour fournir un liquide sous une pression de fonctionnement accrue et à partir duquel le liquide est reçu dans le flux de gaz respiratoire de manière distale, le conduit de liquide (20) étant couplé distalement à un dispositif de pulvérisation (30) comprenant une plaque de pulvérisation en céramique (60) avec au moins un canal de pulvérisation (66) qui possède une section de passage inférieure à environ cinq micromètres, et tel qu'au moins un canal de pulvérisation reçoit le liquide, au moins pendant le fonctionnement, depuis le conduit de liquide sous une pression accrue et permet de délivrer au moins un jet de brouillard dans le flux de gaz respiratoire, **caractérisé par le fait que** le tube endotrachéal comprend un tube d'entrée (17) avec un port à travers lequel le conduit de liquide (20) peut être reçu, ce tube d'entrée continuant dans la lumière de ventilation (13) du tube endotrachéal et s'ouvrant à une distance de 10 centimètres en amont de l'extrémité externe distale (16) du tube endotrachéal, en ce qu'au moins une extrémité extérieure du tube d'admission est reçue par un dispositif de centrage (18) qui permet de centrer au moins l'extrémité extérieure du tube d'admission de manière sensiblement coaxiale dans la lumière de ventilation (13), en ce que le conduit de liquide est fourni par un cathéter d'atomisation individuel (20) comprenant un corps de tige allongé dans lequel une lumière de liquide s'étend axialement, ayant une extrémité extérieure distale qui se connecte au dispositif de pulvérisation (30), et en ce que le cathéter d'atomisation est reçu de manière interchangeable dans le tube endotrachéal (10).

2. Dispositif selon la revendication 1, **caractérisé par le fait qu'**au moins la partie distale du conduit de liquide (20) s'étend à l'intérieur du tube endotrachéal (10) et en particulier se termine au moins substantiellement concentriquement à l'intérieur de celui-ci.

3. Dispositif selon la revendication 1 ou 2, **caractérisé par le fait que** la plaque de pulvérisation céramique (60) comprend une couche de nitrure de silicium (62) dans laquelle l'ouverture de pulvérisation (66) s'étend sur toute l'épaisseur de la couche, que la couche de nitrure de silicium s'étend sur un corps de support en silicium (60) avec une cavité de corps (65) qui s'ouvre à la position du canal de pulvérisation sur une surface de celui-ci, et que la cavité de corps permet de maintenir une connexion de liquide à l'extrémité extérieure distale du conduit de liquide (20).

4. Dispositif selon l'une ou plusieurs des revendications précédentes, **caractérisé par le fait qu'**au moins un canal de pulvérisation (66) possède une longueur inférieure à environ cinq micromètres.

5. Dispositif selon l'une ou plusieurs des revendications précédentes, **caractérisé par le fait que** le tube endotrachéal (10) comprend, à proximité de son extrémité externe ouverte distale, un enregistreur d'images optiques pouvant être couplé à un dispositif de traitement d'images et à un écran.

6. Dispositif selon l'une ou plusieurs des revendications précédentes, **caractérisé par le fait que** le tube endotrachéal (10) comprend un tube d'aspiration avec une buse d'aspiration qui s'ouvre à proximité du dispositif de pulvérisation, et que le tube endotrachéal comprend un port avec un raccord de tube pour connecter le tube d'aspiration à un dispositif d'aspiration externe.

7. Dispositif selon l'une ou plusieurs des revendications précédentes, **caractérisé par le fait que** le système de liquide comprend une seringue médicale (40) comprenant un récipient avec un volume pour le liquide dans lequel un corps de piston (45) activable manuellement est mobile, et **par le fait que** le conduit de liquide comprend à l'extrémité extérieure proximale un raccord (25) pour une connexion étanche au liquide et résistante à la pression à une sortie de la seringue médicale.
